# EUROPEAN PATENT APPLICATION

(11) **EP 4 691 506 A1**
(43) Date of publication of application: **11.02.2026**
(21) Application number: 24781224.1
(22) Date of filing: 27.03.2024
(51) Int. Cl.: A61L 27/60, A61L 27/12, A61L 27/14, A61L 27/20, A61L 27/54, A61L 27/36

(54) **FILLER COMPOSITION COMPRISING NUCLEIC ACID AND CALCIUM HYDROXYLAPATITE**

(30) Priority: 28.03.2023 KR 20230040602
(71) Applicant: Pharma Research Bio Co., Ltd., Gangneung-si, Gangwon-do 25451 (KR)
(72) Inventor: KIM, Heung Joo, Seongnam-si, Gyeonggi-do 13453 (KR); WON, Chee-Youb, Seongnam-si, Gyeonggi-do 13453 (KR); CHOI, Bok Ryul, Seongnam-si, Gyeonggi-do 13453 (KR); PARK, Su Jin, Seongnam-si, Gyeonggi-do 13453 (KR); KIM, Seo Young, Seongnam-si, Gyeonggi-do 13453 (KR)
(74) Representative: Cabinet Beau de Loménie
(86) International application number: PCT/KR2024/003873
(87) International publication number: WO 2024/205234

(57) **Abstract**

Provided is a filler composition comprising nucleic acid and calcium hydroxylapatite.

## Description

### [Technical Field]

The present disclosure relates to a filler composition comprising nucleic acid and calcium hydroxylapatite.

### [Background Art]

With the growing interest in physical appearance, including the face or body, etc., the number of cosmetic procedures aimed at improving skin conditions and compensating for imperfections in appearance is increasing. In particular, there is a growing interest in cosmetic procedures aimed at improving skin wrinkles or other skin appearance conditions. In recent years, methods of injecting substances similar to skin tissue, so-called fillers, into a desired body area are frequently performed both for cosmetic purposes and for medical purposes to replace damaged living tissues. In this regard, fillers play a role in correcting skin imperfections, especially improving the appearance of the skin by adding volume beneath the skin that has been lost due to wrinkles or wounds on the face.

In general, filler compositions with cross-linked hyaluronic acid as a main component are widely used, but there are disadvantages in that they are difficult to inject into the skin due to their high viscosity and low elasticity, and even though the filler compositions are injected into the skin, they do not maintain the injected form (shape) for a long time and have a short shape retention period. In addition, a synthetic polymer, carboxymethyl cellulose (CMC), is often added in order to prepare a long-lasting skin filler composition that exhibits low extrusion force while having improved elasticity and viscosity that provide high volumizing ability (Korean Patent Publication No. 10-2018-0038753). However, carboxymethyl cellulose (CMC) has the problem of poor biocompatibility, which may cause inflammation when subcutaneously administered to animals and may cause fibrosarcoma at the injection site when injected repeatedly.

The development of a filler composition that has excellent biocompatibility and reasonably long-lasting persistence, and provides an effective volumizing capacity is still insufficient, and accordingly, the development of an improved filler composition is still required.

### [Disclosure]

### [Technical Problem]

The present inventors found that a filler composition comprising nucleic acid and calcium hydroxylapatite has excellent biocompatibility and effective volumizing capacity based on excellent physical properties, thereby completing the present disclosure.

### [Technical Solution]

One object of the present disclosure is to provide a filler composition comprising nucleic acid and calcium hydroxylapatite (CaHAp).

### [Advantageous Effects]

A filler composition comprising nucleic acid and calcium hydroxylapatite according to the present disclosure has excellent biocompatibility and effective volumizing capacity.

### [Brief Description of the Drawing]

FIG. 1 shows the appearance according to concentrations of nucleic acid and calcium hydroxylapatite;
FIG. 2 shows the results of measuring injection pressure according to concentrations of nucleic acid and calcium hydroxylapatite;
FIG. 3 shows the results of measuring viscoelasticity according to concentrations of nucleic acid and calcium hydroxylapatite;
FIG. 4 shows the results of comparative evaluation of physicochemical properties between a composition comprising nucleic acid and calcium hydroxylapatite and a composition comprising carboxymethyl cellulose and calcium hydroxylapatite; and
FIG. 5 shows the results of comparative evaluation of biocompatibility between the composition comprising nucleic acid and calcium hydroxylapatite and the composition comprising carboxymethyl cellulose and calcium hydroxylapatite.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

One aspect of the present disclosure provides a filler composition comprising nucleic acid and calcium hydroxylapatite.

The nucleic acid of the present disclosure plays a role in improving the function of the skin itself, as it stimulates formation of the extracellular matrix (ECM), which is an intercellular component, and activates the skin healing ability in the human body, thereby recovering the aged and atrophied regenerability of the skin. The nucleic acid of the present disclosure may be a polynucleotide (PN), a polydeoxyribonucleotide (PDRN), or a mixture thereof, and may be used interchangeably with terms such as 'DNA fragment mixture', 'DNA fraction', etc.

As used herein, the term "polynucleotide" is referred to as "PN" and may refer to a DNA or RNA strand, which is a polymer of nucleotides in which nucleotide units (monomers) are covalently linked to form a chain, and as used herein, the term "polydeoxyribonucleotide" is also referred to as "PDRN" and may refer to, but is not limited to, a type of low molecular weight DNA complex having a specific standard molecular weight. For example, the polynucleotide may have a relatively longer length or larger molecular weight of nucleic acid, as compared to polydeoxyribonucleotide, and may be used as a medical device raw material by exhibiting cell adhesion, lubricating, and buffering effects as a role of a physical support, and the polydeoxyribonucleotide may be used as a raw material for a medicinal product for cell proliferation and tissue regeneration, but is not limited thereto.

In an embodiment according to any one of the aforementioned embodiments, the nucleic acid of the present disclosure may have a molecular weight of about 1 kDa to about 100,000 kDa, about 5 kDa to about 50,000 kDa, about 50 kDa to about 10,000 kDa, or about 50 kDa to about 1,500 kDa.

In an embodiment according to any one of the aforementioned embodiments, the nucleic acid of the present disclosure may be obtained from the testes or semen of fish by extraction. Specifically, the fish may be of the *Salmonidae* family. More specifically, the fish may be salmon or trout, but is not limited thereto.

In the present disclosure, "calcium hydroxylapatite (CaHAp)" is a substance widely used for correcting creases and restoring lost volume. It may be used primarily for removing wrinkles in the facial area and improving facial contours, but is not limited thereto. In addition, the term may be used interchangeably with calcium hydroxyapatite, CaHA, or CaHAp.

The present inventors found that when nucleic acid and calcium hydroxylapatite are included, unlike the general compositions of existing fillers, they may function as an excellent filler, thereby completing the present disclosure.

The composition of the present disclosure may comprise nucleic acid and calcium hydroxylapatite in specific amounts.

In one embodiment, the amount of the nucleic acid comprised in the composition of the present disclosure may be 1% by weight to 5% by weight, based on the total weight of the composition, and the amount of calcium hydroxylapatite disclosure may be 5% by weight to 30% by weight, based on the total weight of the composition, but are not limited thereto.

In an embodiment according to any one of the aforementioned embodiments, the weight ratio of the nucleic acid to the calcium hydroxylapatite comprised in the composition of the present disclosure may be 1:1 to 1: 30, but is not limited thereto. In an embodiment according to any one of the aforementioned embodiments, the nucleic acid comprised in the composition of the present disclosure may be comprised in an amount of 1% by weight to 5% by weight, based on the total weight of the composition.

Specifically, the nucleic acid comprised in the composition of the present disclosure may be comprised in an amount of about 1% by weight to 5% by weight, about 1% by weight to 4% by weight, about 1% by weight to 3% by weight, about 1% by weight to 2% by weight, about 2% by weight to 5% by weight, about 2% by weight to 4% by weight, about 2% by weight to 3% by weight, about 3% by weight to 4% by weight, about 3% by weight to 5% by weight, or about 4% by weight to 5% by weight, based on the total weight of the composition, but is not limited thereto.

In an embodiment according to any one of the aforementioned embodiments, the calcium hydroxylapatite comprised in the composition of the present disclosure may be comprised in an amount of 5% by weight to 30% by weight, based on the total weight of the composition.

Specifically, the calcium hydroxylapatite comprised in the composition of the present disclosure may be comprised in an amount of about 5 to 30% by weight, about 5 to 25% by weight, about 5 to 20% by weight, about 5 to 15% by weight, about 5 to 10% by weight, about 10 to 30% by weight, about 10 to 25% by weight, about 10 to 20% by weight, or about 10 to 15% by weight, based on the total weight of the composition, but is not limited thereto.

In an embodiment according to any one of the aforementioned embodiments, the weight ratio of the nucleic acid to the calcium hydroxylapatite comprised in the composition of the present disclosure may be 1:1 to 1: 30.

Specifically, the weight ratio of the nucleic acid to the calcium hydroxylapatite comprised in the composition of the present disclosure may be about 1: about 30, about 2: about 30; about 3: about 50, about 5: about 30, about 7: about 30, about 10: about 30, about 15: about 30, about 1: about 1, about 1: about 2, about 1: about 3, about 1: about 4, about 1: about 5, about 1: about 6, about 1: about 7, about 1: about 8, about 1: about 9, about 1: about 10, about 1: about 11, about 1: about 12, about 1: about 13, about 1: about 14, about 1: about 15, about 1: about 20, about 1: about 25, or about 1: about 30, but is not limited thereto.

The term "about" comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value. In still another example, the term "about" refers to a range comprising ±0.5, ±0.4, ±0.3, ±0.2, ±0.1, etc., but is not limited thereto.

The filler composition according to the present disclosure may not comprise hyaluronic acid or carboxymethyl cellulose.

Hyaluronic acid fillers are composed of polysaccharides that are similar to the components of the human body, and thus have fewer side effects such as skin hypersensitivity, etc., and they are easy to apply and remove, have excellent viscoelasticity, maintain moisture in the skin, and maintain volume and elasticity of the skin, and for this reason, they are the most widely used fillers at present. Recent research has been actively conducted to extend the duration by inducing cross-linking of hyaluronic acid to increase its particle size and molecular weight. However, since the duration is relatively short at 6 months to 12 months, there is the inconvenience of having to repeat the procedure in a short period of time.

Fillers using synthetic polymers are decomposed very slowly in the human body, and thus they are evaluated and used as semi-permanent fillers, as compared to hyaluronic acid fillers, which are absorbable fillers. Synthetic polymer fillers are fillers in the form of microspheres, and must be administered after being suspended in a gel carrier such as carboxymethylcellulose (CMC), etc. Depending on the average particle size or size distribution of the microspheres, clogging often occurs when injected into the skin, requiring replacement with a thicker needle. In addition, since it takes 6 to 8 weeks to recover after being injected into the skin, there is a disadvantage in that synthetic polymer fillers are not as satisfying as hyaluronic acid fillers showing immediate effects after the procedure.

The filler composition according to the present disclosure may be used for repairing the skin tissue because it has excellent biocompatibility.

Specifically, the filler composition of the present disclosure is a composition comprising a biocompatible material, nucleic acid, i.e., polynucleotide (PN) or polydeoxyribonucleotide (PDRN). Since nucleic acid is known to have a similar base sequence ratio to the human body, it has high biocompatibility for the human body, as compared to existing carboxymethyl cellulose having low biocompatibility, which may cause inflammation when subcutaneously administered to an animal, and may cause fibrosarcoma at the injection site when repeatedly injected. Moreover, the nucleic acid of the present disclosure plays a role in improving the function of the skin itself, as it stimulates formation of extracellular matrix (ECM) which is an intercellular component and activates the skin healing ability in the human body, thereby recovering the aged and atrophied regenerability of the skin. In addition, since the nucleic acid acts as a physical support due to its high chain length and molecular weight, it has the advantage of being able to maintain the dispersion of calcium hydroxylapatite particles well.

The filler composition according to the present disclosure may be for compensating for the volume loss of calcium hydroxyapatite filler.

In addition, the filler composition according to the present disclosure may be for skin injection for wrinkle improvement and shape formation of the skin.

Specifically, the filler composition may be used to improve, but is not limited to, skin wrinkles, glabellar lines, nasolabial folds, chin folds, marionette lines, jawline, buccal commissure, perioral wrinkles, crow's feet, skin depressions, scars, temples, subdermal support of the eyebrows, cheek and cheek fat pads, tear troughs, nose, lips, cheeks, chin, perioral regions, suborbital regions, and facial asymmetry.

The filler composition according to the present disclosure may further comprise one or more compounds selected from the group consisting of anesthetic agents, polyols, vitamins, amino acids, metals, antioxidants, and mineral salts. Specifically, the polyol may be glycerin, but is not limited thereto. The polyol may be comprised in the filler composition in an amount of about 0.01% by weight to about 30% by weight, about 0.5% by weight to about 25% by weight, about 1% by weight to about 20% by weight, about 1% by weight to about 30% by weight, about 5% by weight to about 25% by weight, about 10% by weight to about 20% by weight, or about 15% by weight, based on the total weight of the composition, but is not limited thereto.

The filler composition according to the present disclosure may comprise a buffer, for example, a phosphate buffer, in order to adjust pH. Since the filler composition of the present disclosure is intended for insertion into the human body, the pH is generally in the range of 6.5 to 7.5, specifically, in the range of 6.8 to 7.4, but is not limited thereto. In addition, its osmolality is about 200 mOsmol/l to about 400 mOsmol/l, specifically, about 280 mOsmol/l to about 330 mOsmol/l, but is not limited thereto.

The filler composition according to the present disclosure provides a filler composition exhibiting an appropriate range of elastic modulus or viscous modulus that is injectable into a target tissue.

As used herein, the term "elasticity" refers to a property as a solid when a force is applied to an object, that is, a property in which the shape changes when a force is applied but returns to its original shape when the force is removed. The elasticity of the filler refers to the degree to which a desired shape is maintained under the skin tissue, and as the elasticity is higher, the original treated shape is maintained for a longer time. The elasticity is represented by a storage elastic modulus (G': elastic modulus, storage modulus), and the unit is Pascal (Pa). In addition, as used herein, the term "viscosity" refers to exhibiting a property as a liquid, that is, a viscous flow which is a resistance to the flow. The viscosity may be represented by a loss viscous modulus (G": viscous modulus, loss modulus), and the unit is Pascal (Pa).

As the elasticity of the filler composition is higher, it forms a particle shape, and feels a little firmer, which is better for providing volume, and the filler composition has the characteristic of maintaining its shape well after one procedure. In addition, as the elasticity is higher, the filler composition has the characteristic of having a longer duration. However, when the elasticity is excessively high, there is the problem that the surface is uneven and layers are formed on the skin depending on the skill level.

When the viscosity of the filler composition is high, it forms a sol shape and is soft and squishy, and thus it naturally settles into the skin. However, as the filler composition is relatively less firm, there is a limit to creating a definite volume.

Therefore, it is important to inject the desired filler into a target tissue by controlling elasticity and viscosity.

In one embodiment, the filler composition of the present disclosure may exhibit an elastic modulus of about 20 Pa to about 45,000 Pa. For example, the composition exhibits an elastic modulus of about 20 Pa to about 45,000 Pa, about 30 Pa to about 30,000 Pa, about 40 Pa to about 20,000 Pa, about 50 Pa to about 10,000 Pa, about 100 Pa to about 8,000 Pa, about 500 Pa to about 7,000 Pa, about 1,000 Pa to about 6,000 Pa, about 1,500 Pa to about 5,000 Pa, or about 2,000 Pa to about 4,000 Pa. In another embodiment, the filler composition of the present disclosure may exhibit an elastic modulus of about 20 Pa or more, about 25 Pa or more, about 50 Pa or more, about 75 Pa or more, about 100 Pa or more, about 125 Pa or more, about 150 Pa or more, about 175 Pa or more, about 200 Pa or more, about 300 Pa or more, about 400 Pa or more, about 500 Pa or more, about 600 Pa or more, about 700 Pa or more, about 800 Pa or more, about 900 Pa or more, about 1,000 Pa or more, about 1,200 Pa or more, about 1,400 Pa or more, about 1,600 Pa or more, about 1,800 Pa or more, about 2,000 Pa or more, about 2,500 Pa or more, about 3,000 Pa or more, about 3,500 Pa or more, about 4,000 Pa or more, about 4,500 Pa or more, about 5,000 Pa or more, about 5,500 Pa or more, about 6,000 Pa or more, about 6,500 Pa or more, about 7,000 Pa or more, about 7,500 Pa or more, about 8,000 Pa or more, about 8,500 Pa or more, about 9,000 Pa or more, about 9,500 Pa or more, about 10,000 Pa or more, about 20,000 Pa or more, about 30,000 Pa or more, about 40,000 Pa or more, or about 45,000 Pa or more.

Commonly used filler compositions have an elastic modulus in the range of about 20 Pa or more to about 45,000 Pa or less. The elastic modulus within the above range enables a homogeneous injectable composition and allows the nucleic acid and/or calcium hydroxylapatite to remain in a homogeneous state in the tissue within the skin after administration, thereby avoiding differences in effectiveness from site to site. In addition, when injected into the body, the tissue repair effect may be evenly distributed.

When the filler composition has an elasticity range of about 20 Pa or less, there are problems that it does not retain its injected form (shape) for a long time and has a short shape retention period. When the elasticity range is too high, such as about 45,000 Pa or more, extrusion from a syringe may be difficult when injected into the skin through the syringe.

In one embodiment, the filler composition of the present disclosure may exhibit a viscous modulus of about 1 Pa to about 8,000 Pa. For example, the composition exhibits a viscous modulus of about 3 Pa to about 7,500 Pa, about 5 Pa to about 7,000 Pa, about 10 Pa to about 6,500 Pa, about 20 Pa to about 6,000 Pa, about 30 Pa to about 5,000 Pa, about 40 Pa to about 3,000 Pa, about 50 Pa to about 2,000 Pa, or about 60 Pa to about 1,000 Pa. In another embodiment, the filler composition of the present disclosure may exhibit a viscous modulus of about 1 Pa or more, about 2 Pa or more, about 3 Pa or more, about 5 Pa or more, about 10 Pa or more, about 20 Pa or more, about 30 Pa or more, about 40 Pa or more, about 50 Pa or more, about 100 Pa or more, about 200 Pa or more, about 300 Pa or more, about 500 Pa or more, about 700 Pa or more, about 900 Pa or more, about 1,000 Pa or more, about 1,500 Pa or more, about 2,000 Pa or more, about 2,500 Pa or more, about 3,000 Pa or more, about 3,500 Pa or more, about 4,000 Pa or more, about 4,500 Pa or more, about 5,000 Pa or more, about 5,500 Pa or more, about 6,000 Pa or more, about 6,500 Pa or more, about 7,000 Pa or more, about 7,500 Pa or more, or about 8,000 Pa or more.

Commonly used filler compositions have a viscous modulus in the range of about 1 Pa or more to about 8,000 Pa or less, and the viscous modulus within the above range enables formation of a soft, pliable, and suitable sol shape, which allows the filler composition to settle naturally in the tissue within the skin after administration.

When the filler composition has the viscosity range of about 1 Pa or less, the filler composition settles in the skin but has a poor ability to form its own shape, and thus it does not play a role as a filler to create volume. When the viscosity range is too high, such as about 8,000 Pa or more, there is difficulty in injecting an accurate amount because excessive force may be applied due to difficult extrusion from a syringe when injected into the skin through the syringe, and it is difficult to precisely control the amount of injection, which may cause side effects.

The filler composition of the present disclosure provides an injectable composition. The term "injectable" refers to a material having the properties necessary to administer the composition into a dermal region of an individual using an injection device with a fine needle. The term "fine needle" may refer to, for example, a needle of 27 gauge or smaller, and the fine needle may be appropriately selected by a person skilled in the art according to its purpose by controlling the particle size of the nucleic acid and calcium hydroxylapatite.

The filler composition of the present disclosure is injectable through a fine needle. For example, the filler composition is injectable through a needle of about 27 gauge, about 30 gauge, or about 32 gauge. Specifically, the filler composition is injectable through a needle of 22 gauge or less, 27 gauge or less, 30 gauge or less, or 32 gauge or less. For another example, the filler composition is injectable through a needle of about 22 gauge to about 35 gauge, about 22 gauge to about 34 gauge, about 22 gauge to about 33 gauge, about 22 gauge to about 32 gauge, about 22 gauge to about 27 gauge, or about 27 gauge to about 32 gauge, but is not limited thereto.

The filler composition of the present disclosure may be injected with an extrusion force of about 60 N, about 55 N, about 50 N, about 45 N, about 40 N, about 35 N, about 30 N, about 25 N, about 20 N, or about 15 N at a speed of 12 mm/min. For example, the filler composition of the present disclosure may be injected through a needle of 27 gauge with an extrusion force of about 60 N or less, about 55 N or less, about 50 N or less, about 45 N or less, about 40 N or less, about 35 N or less, about 30 N or less, about 25 N or less, or about 20 N or less, about 15 N or less, about 10 N or less, or about 5 N or less. Further, the filler composition of the present disclosure may be injected through a needle of 30 gauge with an extrusion force of about 60 N or less, about 55 N or less, about 50 N or less, about 45 N or less, about 40 N or less, about 35 N or less, about 30 N or less, about 25 N or less, about 20 N or less. Further, the filler composition of the present disclosure may be injected through a needle of 32 gauge with an extrusion force of about 60 N or less, about 55 N or less, about 50 N or less, about 45 N or less, about 40 N or less, about 35 N or less, about 30 N or less, about 25 N or less, about 20 N or less, about 15 N or less, about 10 N or less, or about 5 N or less, but is not limited thereto.

As described above, the filler compositions of the present disclosure have a wide range of viscoelasticity and injection pressure, and accordingly, an appropriate filler composition may be selected for use, depending on the purpose of use according to phase characteristics, the intended tissue injection site and preference, etc.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Preparation of Composition

Compositions were prepared using PN (Pharmaresearch, Korea, molecular weight of 50 kDa to 1,500 kDa) and CaHAp (nanografi, Germany) according to the compositions as in Table 1 below. In detail, PBS (1 M potassium phosphate buffer was used as a stock solution), glycerin, D.W, and PN were each quantitatively weighed, mixed and dissolved. CaHAp was quantitatively weighed and added to the solution, and then mixed. Thereafter, the mixture was filled into a glass syringe. The mixture was sterilized at 121°C for 20 minutes.

**[Table 1]**

| **S ample** | **PN (g)** | **CaHAp (g)** | **Glycerin (g)** | **PBS (g)** | **D.W (g)** | **Total (g)** |
|---|---|---|---|---|---|---|
| P0.5_C5 | 0.025 | 0.25 | 0.75 | 0.5 | 3.475 | 5 |
| P0.5_C10 | 0.025 | 0.5 | | | 3.225 | |
| P0.5_C30 | 0.025 | 1.5 | | | 2.225 | |
| P0.5_C45 | 0.025 | 2.25 | | | 1.475 | |
| P0.5_C50 | 0.025 | 2.5 | | | 1.225 | |
| P1_C5 | 0.05 | 0.25 | | | 3.45 | |
| P1_C10 | 0.05 | 0.5 | | | 3.2 | |
| P1_C30 | 0.05 | 1.5 | | | 2.2 | |
| P1_C45 | 0.05 | 2.25 | | | 1.45 | |
| P1_C50 | 0.05 | 2.5 | | | 1.2 | |
| P3_C5 | 0.15 | 0.25 | | | 3.35 | |
| P3_C10 | 0.15 | 0.5 | | | 3.1 | |
| P3_C30 | 0.15 | 1.5 | | | 2.1 | |
| P3_C45 | 0.15 | 2.25 | | | 1.35 | |
| P3_C50 | 0.15 | 2.5 | | | 1.1 | |
| P5_C5 | 0.25 | 0.25 | | | 3.25 | |
| P5_C10 | 0.25 | 0.5 | | | 3 | |
| P5_C30 | 0.25 | 1.5 | | | 2 | |
| P5_C45 | 0.25 | 2.25 | | | 1.25 | |
| P5_C50 | 0.25 | 2.5 | | | 1 | |
| P7_C5 | 0.35 | 0.25 | | | 3.15 | |
| P7_C10 | 0.35 | 0.5 | | | 2.9 | |
| P7_C30 | 0.35 | 1.5 | | | 1.9 | |
| P7_C45 | 0.35 | 2.25 | | | 1.15 | |
| P7_C50 | 0.35 | 2.5 | | | 0.9 | |

### Example 2. Evaluation of Appearance According to Contents of CaHAp + PN

It was intended to evaluate the appearance according to the contents of CaHAp + PN using the compositions in Table 1.

In detail, the appearance of the mixture in the glass vial was observed.

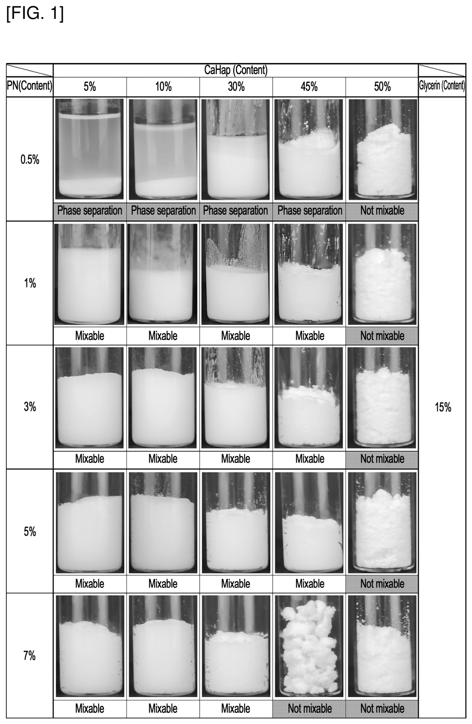

As a result, as shown in FIG. 1, when the content of PN was 0.5% by weight, phase separation occurred and mixing was impossible, and when the content of CaHAp was 50% by weight, mixing was impossible. In addition, when the content of PN was 7% by weight and the content of CaHAp was 5% by weight to 30% by weight, mixing was possible, but the gel crumbled and did not exhibit proper appearance.

The above results confirmed that, in terms of appearance, when the content of PN was 1% by weight to 5% by weight and the content of CaHAp was 5% by weight to 45% by weight, appropriate appearance was exhibited. The absence of phase separation means that the particle dispersion of calcium hydroxylapatite was well maintained, and thus the particles may serve as a physical support.

### Example 3. Evaluation of Injection Pressure According to Contents of CaHAp + PN

It was intended to evaluate the injection pressure according to the contents of CaHAp + PN using the compositions in Table 1.

In detail, the injection pressure was measured using the conditions and methods as in Table 2 below.

**[Table 2]**

| | |
|---|---|
| **Needle Gauge** | 27G |
| **Injection Speed** | 12 mm/min |

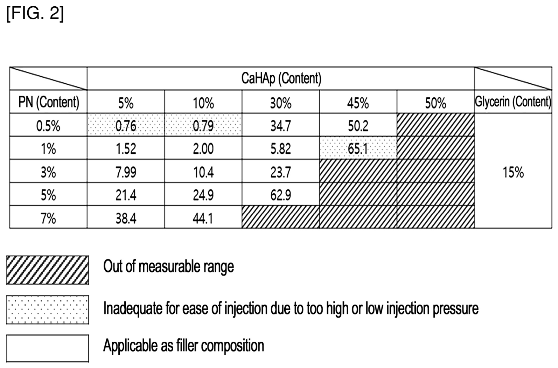

As a result, as shown in FIG. 2, it was confirmed that those in the hatched area were not measurable, but the measurable samples showed a range from a minimum of 0.76 N to a maximum of 65.1 N.

In detail, it was confirmed that when the content of PN was 0.5% by weight and the content of CaHAp was 5% by weight to 10% by weight, a nonsignificant low value that was inadequate for ease of injection was measured, and when the content of PN was 0.5% by weight, and the content of CaHAp was 30% by weight to 50% by weight, liquid and solid phases are separated due to phase separation depending on the content of PN, and thus the injection pressure increased, and as a result, in some ranges, measurement was not possible. It was also confirmed that when the content of CaHAp was 45% by weight to 50% by weight, mixing was impossible in some compositions, and thus the injection pressure was not measurable. However, it was confirmed that when the content of PN was 0.5% by weight to 7% by weight, and the content of CaHAp was in the range of 5% by weight to 50% by weight, the injection pressure in a wide range of, i.e., about 1 N or more to about 65 N or less, applicable as a filler according to each content, was exhibited.

### Example 4. Evaluation of Physicochemical Properties (Viscosity/Elasticity) According to Contents of CaHAp + PN

It was intended to evaluate the physicochemical properties (viscosity/elasticity) according to the contents of CaHAp + PN using the compositions in Table 1.

In detail, the viscosity/elasticity were measured using the conditions and methods as in Table 3 below.

**[Table 3]**

| **Oscillation (Value measured at 1.0 Hz)** | |
|---|---|
| Geometry | Parallel Plate 25 mm |
| Gap | 1.0 mm |
| Initial Frequency | 0.1 Hz |
| Final Frequency | 10 Hz |
| Shear Strain | Fixed at 1.0% |

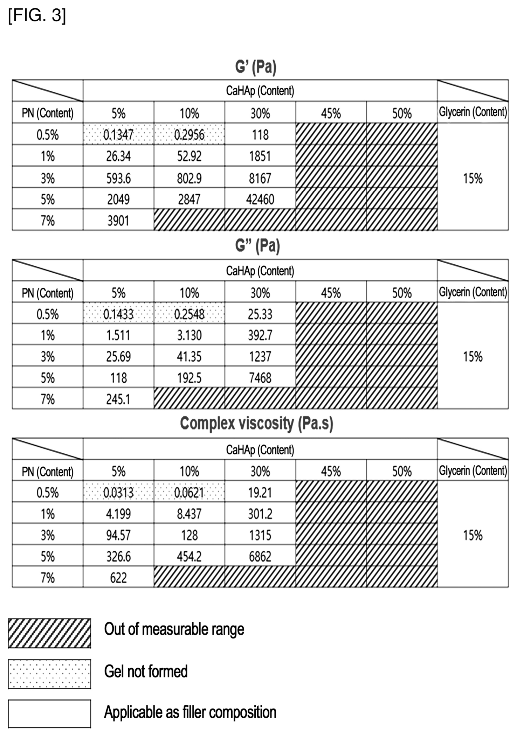

As a result, as shown in FIG. 3, when the content of PN was 0.5% by weight and the content of CaHAp was 5% by weight to 10% by weight, a nonsignificant low value that did not form a gel was measured, and when the content of CaHAp was 45% by weight to 50% by weight, measurement was impossible.

However, when the content of PN was 1% by weight to 5% by weight, measurement was possible up to a content of CaHAp of 5% by weight to 30% by weight, and when the content of PN was 7% by weight, measurement was possible up to a content of CaHAp of 5% by weight.

In detail, it was confirmed that when the content of PN was 0.5% by weight and the content of CaHAp was 5% by weight to 10% by weight, the viscosity and elasticity values were about 0.5 Pa or less, and when the content of PN was 0.5% by weight and the content of CaHAp was 45% by weight to 50% by weight, neither the viscosity nor the elasticity values were measurable.

When the content of PN was 1% by weight to 5% by weight and the content of CaHAp was 5% by weight to 30% by weight, as shown in FIG. 3, the elastic modulus was measured from about 25 Pa or more to about 43,000 Pa or less, and the viscous modulus was measured from about 1.5 Pa or more to about 8,000 Pa or less.

The filler composition of the present disclosure has various properties and injection pressures, suggesting that the filler may be injected according to the purpose by controlling the injection pressure and viscosity/elasticity depending on the site for elasticity and repair in a target tissue.

### Example 5. Comparative Evaluation of Physicochemical Properties (Viscosity/Elasticity) of CaHAp + PN

It was intended to evaluate the physicochemical properties of CaHAp + PN compositions by comparing them with compositions with different components. As a comparative composition, a CaHAp + CMC composition comprising carboxymethyl cellulose (CMC) as a component, instead of PN of the filler composition of the present disclosure, was selected.

The CaHAp + PN composition and CaHAp + CMC composition were prepared according to the compositions as in Table 4 below. 10% by weight of CaHAp was comprised in both compositions, and 3% by weight of PN or CMC was comprised in each composition. In detail, PBS, glycerin, D.W, CMC, and PN were each quantitatively weighed and mixed, and then dissolved. Then, CaHAp was quantitatively weighed and added to each solution, and then mixed. The mixture was filled into a glass syringe, and the mixture was sterilized at 121°C for 20 minutes.

**[Table 4]**

| **Sample** | **PN/CMC (g)** | **CaHAp (9)** | **Glycerin (g)** | **PBS (g)** | **D.W** | **Total (g)** |
|---|---|---|---|---|---|---|
| CaHAp + PN | 0.15 | 0.5 | 0.75 | 0.5 | 3.1 | 5 |
| CaHAp + CMC | 0.15 | 0.5 | 0.75 | 0.5 | 3.1 | 5 |

The viscosity and elasticity of each composition above were measured using the conditions and methods shown in Table 3.

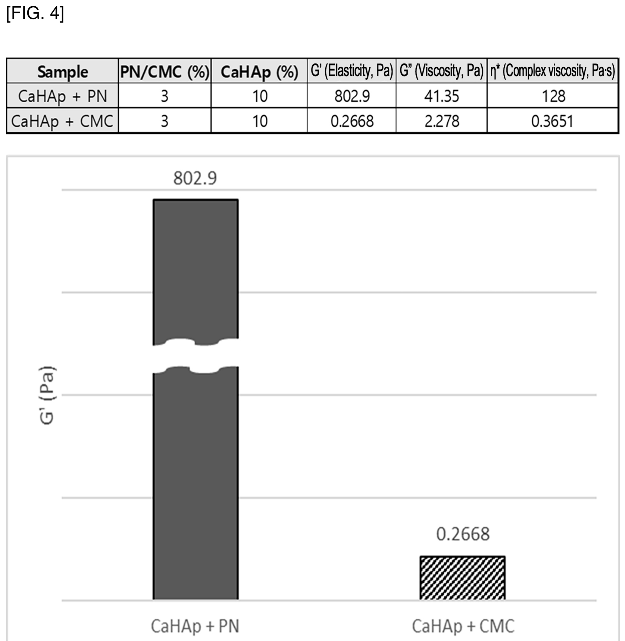

As a result, as shown in FIG. 4, it was confirmed that the elasticity and viscosity of the CaHAp + PN composition were measured higher than those of the CaHAp + CMC composition, experimentally confirming that the properties were improved by use of PN, as compared to use of CMC, when the mixture with CaHAp was prepared. This means that the CaHAp + PN composition has a wide range of properties that may be adjusted according to a desired tissue, based on the improved properties, and thus its application range for tissue repair is significantly expanded.

### Example 6. Evaluation of Biocompatibility of CaHAp + PN

In order to evaluate the biocompatibility of the CaHAp + PN composition, a WST assay was performed on the composition. In addition, for comparative evaluation of the biocompatibility of the composition, the same experiment was performed on the CaHAp + CMC composition, and the measured values were compared.

The CaHAp + PN composition, CaHAp + CMC composition, and control composition were prepared according to the compositions as in Table 5 below, and each was placed in a 50 mL conical tube. After mixing using a vortexer, they were each heated in boiling water at about 68°C. The solution was mixed using a vortexer at 30-minute intervals and then sterilized using an autoclave at 121°C for 20 minutes. Thereafter, the appearance of the solution was observed, and then mixed using a syringe-to-syringe method, and the mixed solution was filled into a 1 mL glass syringe.

**[Table 5]**

| **Sample** | **PN/CMC (g)** | **CaHAp (9)** | **Glycerin (g)** | **PBS (g)** | **D.W** | **Total (g)** |
|---|---|---|---|---|---|---|
| CaHAp + PN | 0.25 | 0.5 | 0.75 | 0.5 | 3.0 | 5 |
| CaHAp + CMC | 0.25 | 0.5 | 0.75 | 0.5 | 3.0 | 5 |
| Base(Control) | - | - | 0.75 | 0.5 | 3.75 | 5 |

As a cell line for the WST assay, the human dermal fibroblast HDF cell line was used. The medium of the stabilized cells (HDF) was removed, the cells were washed with Dulbecco's Phosphate Buffered Saline (DPBS), and the DPBS was also removed. Thereafter, each well was treated with 2 mL of trypsin-EDTA solution and incubated at 37°C for 3 minutes to detach the cells. The medium was mixed in a conical tube to neutralize trypsin, followed by centrifugation at 1,000 rpm for 5 minutes. The supernatant of the solution was discarded, and the medium was added to the cell pellet that had settled to the bottom, and then evenly mixed by pipetting. Thereafter, the cell number was counted using a hemocytometer, and 1x10⁵ cells/well were added to each well of a 96-well plate and incubated at 37°C for 24 hours. In the case of the control group, each well was treated with 100 µL of the sample and incubated at 37°C for 24 hours. The wells incubated for 24 hours were taken out, washed with DPBS, and 10 µL of WST agent (Ez-Cytox) was added to 100 pL of the culture medium, and this solution was dispensed and allowed to react by incubation at 37°C for 1 hour. Thereafter, absorbance was measured at 450 nm using a spectrophotometer. Before measuring the absorbance, the solution was gently shaken for about 1 minute and then measured.

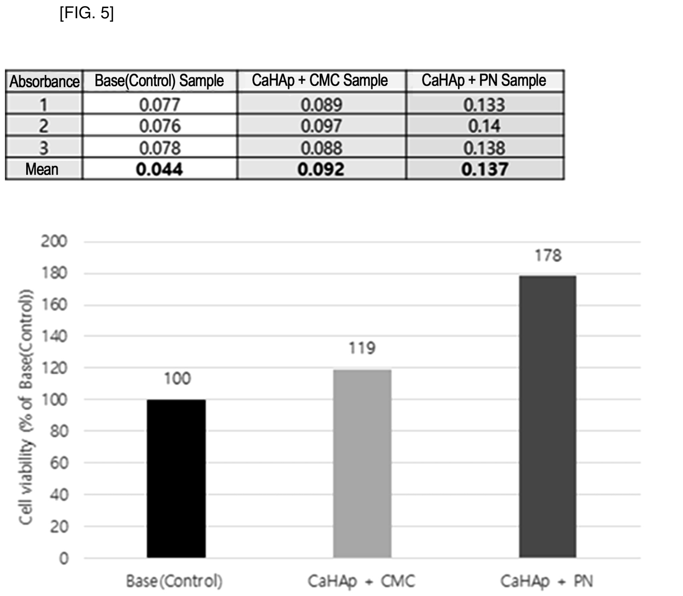

As a result, as shown in FIG. 5, it was confirmed that, in terms of the measured value, the CaHAp + PN composition showed a value 1.5 times higher than the CaHAp + CMC composition, experimentally confirming that the biocompatibility was significantly improved by use of PN, as compared to use of CMC, when the mixture with CaHAp was prepared.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A filler composition comprising nucleic acid and calcium hydroxylapatite (CaHAp), wherein the content of the nucleic acid of the composition is 1% by weight to 5% by weight, based on the total weight of the composition, and the content of the calcium hydroxylapatite is 5% by weight to 30% by weight, based on the total weight of the composition.

2. The filler composition of claim 1, wherein the nucleic acid is polynucleotide, polydeoxyribonucleotide, or a mixture thereof.

3. The filler composition of claim 1, wherein the nucleic acid has a molecular weight of 50 kDa to 10,000 kDa.

4. The filler composition of claim 1, wherein the nucleic acid is separated from the testes or semen of fish.

5. The filler composition of claim 4, wherein the fish is of the *Salmonidae* family.

6. The filler composition of claim 1, wherein a weight ratio of the nucleic acid to the calcium hydroxylapatite is 1:1 to 1: 30.

7. The filler composition of claim 1, not comprising hyaluronic acid or carboxymethyl cellulose.

8. The filler composition of claim 1, wherein the filler composition is used for repairing skin tissue due to excellent biocompatibility.

9. The filler composition of claim 1, wherein the composition is for compensating for the volume loss of calcium hydroxyapatite fillers.

10. The filler composition of claim 1, wherein the composition is for skin injection for wrinkle improvement and shape formation of the skin.

11. The filler composition of any one of claims 1 to 10, wherein the composition is used to improve skin wrinkles, glabellar lines, nasolabial folds, chin folds, marionette lines, jawline, buccal commissure, perioral wrinkles, crow's feet, skin depressions, scars, temples, subdermal support of the eyebrows, cheek and cheek fat pads, tear troughs, nose, lips, cheeks, chin, perioral regions, suborbital regions, and facial asymmetry.

12. The filler composition of claim 1, wherein the composition further comprises one or more compounds selected from the group consisting of anesthetic agents, polyols, vitamins, amino acids, metals, antioxidants, and mineral salts.

13. The filler composition of claim 12, wherein the polyol is glycerin.
